Europäisches Patentamt

European Patent Office

Office européen des brevets

(11) Veröffentlichungsnummer: **0 244 834 B1**

# (12) EUROPÄISCHE PATENTSCHRIFT

(45) Veröffentlichungstag der Patentschrift: **18.03.92**

(21) Anmeldenummer: **87106523.1**

(22) Anmeldetag: **06.05.87**

(51) Int. Cl.⁵: **A61K 35/16**

(54) Verfahren zur Herstellung eines Faktor V-Konzentrats.

(30) Priorität: **09.05.86 DE 3615558**

(43) Veröffentlichungstag der Anmeldung:
**11.11.87 Patentblatt 87/46**

(45) Bekanntmachung des Hinweises auf die
Patenterteilung:
**18.03.92 Patentblatt 92/12**

(84) Benannte Vertragsstaaten:
**AT BE CH DE ES FR GB IT LI LU NL SE**

(56) Entgegenhaltungen:
**EP-A- 0 056 629**

**B.A.L. HURN: "Storage of blood", 1968, Seiten 26-41,136-138, Academic Press Inc., London, GB**

**THE JOURNAL OF BIOLOGICAL CHEMISTRY, Band 256, Nr. 2, 25. Januar 1981, Seiten 1002-1007; W.H. KANE et al.: "Purification and characterization of human coagulation factor V"**

(73) Patentinhaber: **BEHRINGWERKE Aktiengesellschaft**
**Postfach 1140**
**W-3550 Marburg 1(DE)**

(72) Erfinder: **Heimburger, Norbert, Prof.Dr.**
**Sonnenhang 10**
**W-3550 Marburg(DE)**
Erfinder: **Kumpe, Gerhardt**
**Aspherfeld 14**
**W-3552 Wetter(DE)**
Erfinder: **Wormsbächer, Wilfried**
**Blumenweg 9**
**W-3575 Kirchhain(DE)**

(74) Vertreter: **Klein, Otto, Dr. et al**
**Hoechst AG Zentrale Patentabteilung Postfach 80 03 20**
**W-6230 Frankfurt am Main 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BIOCHEMISTRY, Band 15, Nr. 9, 1976, Seiten 1830-1838; C.M. SMITH et al.: "The activation of factor V by factor Xa or alpha-chymotrypsin and comparison with thrombin and RVV-V action. An improved factor V isolation procedure"

CHEMICAL ABSTRACTS, Band 82, Nr. 1, 6. Januar 1975, Seite 205, Zusammenfassung Nr. 2203k, Columbus, Ohio, US; C.L. JANSSEN et al.: "Conditions for stabilization and determination of activated factor V", & THROMB. RES. 1974, 5(3), 315-25

**Beschreibung**

Die Erfindung betrifft ein Verfahren zur Herstellung eines Faktor V-Konzentrats aus humanem Blutplasma oder einer Fraktion aus Blutplasma, die ein Antikoagulans enthalten.

Zu dem biologischen Phänomen der Blutstillung tragen die Gefäße, die Blutplättchen und plasmatische Faktoren bei. Der primäre Wundverschluß entsteht durch das synergistische Zusammenwirken von Gefäßen und Blutplättchen, die zu dem sogenannten primären Wundverschluß führen, der dann durch die plasmatische Gerinnung, die zur Bildung von Fibrin führt, verfestigt wird. Die Blutplättchen bilden die Reaktionsoberfläche für den Ablauf der plasmatischen Gerinnung, an der 13 plasmatische Faktoren, die Inhibitoren nicht eingerechnet, teilhaben. Bei diesen plasmatischen Faktoren handelt es sich um Proteine, vorwiegend Glykoproteine, die ihrer Wirkung nach vorwiegend Serin-Proteasen sind. Diese zirkulieren in Form inaktiver Vorstufen im Blut und werden auf Reize hin, wie sie eine Verletzung darstellen, aktiviert. Die Aktivierung erfolgt nach dem Prinzip einer limitierten Proteolyse, wobei der in der Gerinnungskaskade vorausgehende Faktor den jeweils auf ihn folgenden nach dem Prinzip einer Enzym-Substrat-Reaktion aktiviert. Essentiell für die Aktivierung sind Phospholipide, wie sie in Form eines Gemisches, dem sogenannten Plättchenfaktor 3 (PF 3), in den Plättchen gefunden werden, und Calciumionen. Daneben sind sogenannte Akzeleratoren von Bedeutung, Proteine, die den Ablauf der Gerinnung zu beschleunigen vermögen. Zu den Akzeleratoren, die in Verbindung mit Phospholipiden und Calcium wirken, indem sie sogenannte Aktivierungskomplexe bilden, gehören die Faktoren V und VIII (F V und F VIII).

F V ist ein $\beta$-Globulin mit einem Molekulargewicht von 330.000 Dalton, das über eine limitierte Proteolyse von Thrombin aktiviert und durch Protein $C_a$ inaktiviert wird. Dabei entstehen spezifische Aktivierungspeptide.

Die Wirkung von F V als Akzelerator ist darauf zurückzuführen, daß es die Wechselwirkung zwischen F X a und Prothrombin in Gegenwart von Calcium und Plättchenphospholipiden so intensiviert, daß die Thrombinbildung etwa 1000-fach beschleunigt wird. Den Komplex aus den Faktoren X a, Phospholipiden, Calcium und F V a bezeichnet man als Prothrombin-Aktivator oder Prothrombinase. Entsprechend der Akzeleratorwirkung des F V führt eine Verminderung dieses Gerinnungsfaktors zu einer Verlangsamung der Thrombinbildung und damit der Gerinnung und als biologischer Konsequenz zu einem erhöhten Blutungsrisiko.

Alle Gerinnungsfaktoren, unabhängig von ihrem enzymatischen Charakter, sind vergleichsweise sehr labil und denaturierungsempfindlich, jedenfalls in der Form, wie sie üblicherweise aus antikoaguliertem Plasma gewonnen werden. Im besonderen Maße gilt das für den F VIII und den F V, die beiden Akzeleratorproteine.

Von der Instabilität des Faktors V macht man bei der Herstellung von F V-Mangelplasma Gebrauch: Human-Citratplasma, das in einem Wasserbad bei 37°C inkubiert wird, verliert innerhalb von 24 Stunden seine F V-Aktivität, während alle anderen Faktoren stabil bleiben.

Angeborene und erworbene F V-Mangelzustände sind relativ selten. Trotzdem gibt es eine Reihe von Indikationen, wo ein F V-Konzentrat indiziert erscheint. Bedingt durch die Labilität und Denaturierbarkeit ist bis heute jedoch ein solches Konzentrat nicht verfügbar. Das ist ganz im Gegensatz zu den anderen Gerinnungsfaktoren, die alle in Form von Konzentraten für die Therapie zur Verfügung stehen. Das verdeutlicht die besondere Instabilität dieses Gerinnungsfaktors, die ihren Niederschlag in der Bezeichnung "labiler Faktor" für Faktor V gefunden hat.

Aus diesen Gründen bestand ein Bedürfnis nach einem Verfahren zur Gewinnung von F V aus Vollblut, das die Stabilität dieses Faktors während der Abnahme, Lagerung und Aufarbeitung des Blutes in Gegenwart von Antikoagulantien gewährleistet. Eine solche Stabilisierung von F V würde die Herstellung eines stabilen F V-Plasmas für gerinnungsanalytische Untersuchungen sowie von F V-haltigem Plasma für die Substitution oder als Ausgangsmaterial für die Herstellung eines F V-Konzentrates ermöglichen.

Aufgabe der Erfindung ist die Herstellung von Faktor V-Konzentrat.

Obwohl der F V leicht denaturierbar ist, ist er bereits aus Plasma in aktiver Form isoliert worden. Das war möglich, indem man frischem Citratplasma, aus dem die Prothrombinfaktoren entfernt worden waren, synthetische Protease-Inhibitoren als Schutz gegen einen enzymatischen Abbau des F V zusetzte. Für die Herstellung von einem Standardplasma und auch für die Reindarstellung von F V kommen solche Zusätze nicht in Betracht, da sie toxisch sind und die Wechselwirkung des F V mit anderen Gerinnungsfaktoren blockieren.

Es ist auch bereits bekannt, Faktor V durch Chromatographie an einem Anionenaustauscher aus Plasma zu gewinnen, aus dem das Kryopräzipitat abgetrennt wurde. Nachteilig an diesem Verfahren ist die niedrige Ausbeute.

Kane W. H. et al., THE JOURNAL OF BIOLOGICAL CHEMISTRY, Band 256, Nr. 2, 1981, Seiten 1002 - 1003 beschreibt ein Verfahren zur Isolierung von Faktor V aus einer ein Antikoagulans enthaltenden Fraktion aus humanem Blutplasma unter Verwendung von Calciumchlorid. Gemäß diesem Verfahren wird der im Citratplasma enthaltene Faktor V durch Adsorption auf Bariumcitrat in Gegenwart von 11 % Polyethylenglycol teilweise gereinigt und dann durch DEAE-[R]Sepharose- und [R]Ultrogel AcA 34-Chromatographie isoliert. Für die chromatographische Trennung des Faktors V werden Äquilibrier- bzw. Elutions- Lösungsmittel verwendet, die 5 mmol/l Calciumchlorid enthalten, da dieses den Faktor V stabilisiert.

Weiss. H. J. THROMBOS. DIATHES, HAEMORRH, Band 14, Nr. 32, 1965, Seiten 32 - 51 weist darauf hin, daß die Labilität des Faktors V mit der Konzentration der Antikoagulantien steigt, daß Calcium das Plasmakation zu sein scheint, welches den Faktor V gegen den Verlust der Aktivität in vitro schützt, und daß in Abhängigkeit von der Art des verwendeten Antikoagulansplasma verschiedene divalente Kationen den Faktor V stabilisieren können.

Blombäck B., NATURE, Band 198, Nr. 4883 zeigt den stabilisierenden Effekt von Calcium (0,1 mM) auf die Aktivität von Faktor V in partiell gereinigten Plasmafraktionen aus Citratplasma.

Überraschenderweise wurde gefunden, daß der Verlust an Faktor V-Aktivität bei einer Gewinnung aus Plasma eine Funktion der Citratkonzentration ist, wenn Citrat als Antikoagulans verwendet wird. Während F V bei 20°C und 5 mmol/l Citrat relativ stabil ist, kommt es mit steigenden Citratkonzentrationen zu einer Denaturierung. Eine Citratkonzentration von 5 mmol/l konnte nicht unterschritten werden, wenn eine Gerinnung vermieden werden sollte.

Überraschenderweise wurde weiterhin gefunden, daß ein Aktivitätsverlust durch eine Zugabe zweiwertiger Kationen, vorzugsweise Calciumionen verringert werden kann. Während die F V-Aktivität in Citratplasma, das üblicherweise Tri-Na-Citrat in einer Konzentration von 11 mmol/l enthält, bei 37°C nach 21 Stunden auf Werte nache 0 % der Norm abfällt, bleiben 35 % der Aktivität erhalten, wenn eine solche Lösung 2,5 mmol/l Calciumionen, und 70 % wenn sie 5 mmol/l Calciumionen enthält.

Gegenstand der Erfindung ist ein Verfahren zur Herstellung eines Konzentrats von Gerinnungsfaktor F V aus einem ein Antikoagulans enthaltenden humanen Blutplasma oder Blut, dadurch gekennzeichnet, daß man einem solchen Plasma oder Blut ein Salz von Calcium, Magnesium oder Mangan in einer Menge zusetzt, daß eine Konzentration an zweiwertigen Kationen dieser Metalle von mindestens 2 mmol/l aber kleiner als die der Konzentration an Antikoagulans äquivalenten Konzentration erreicht wird und auf bekannte Weise ein F V-Konzentrat gewinnt.

Vorzugsweise ist ein solches Metallsalz ein lösliches Salz des Calciums, besonders Calciumchlorid oder -azetat.

Die Konzentration an zweiwertigen Kationen ist mindestens 2 mmol/l. Die Wirkung muß nicht stetig mit der Konzentration ansteigen, sondern kann ein Maximum durchlaufen. Die Konzentration darf selbstverständlich nicht so groß sein, daß die Mischung koaguliert.

Antikoagulantien sind vor allem Salze der Zitronensäure, Heparin, Phosphat- oder Citratdextrose oder Mischungen von diesen.

Die zweiwertigen Kationen müssen nicht unmittelbar nach Entnahme des Blutes zugesetzt werden. Es wird auch dann noch ein vorteilhafter Effekt erzielt, wenn man diese bis zu 12 Stunden nach der Blutentnahme zusetzt, speziell dann, wenn das Plasma so lange bei 3-5°C gelagert wurde. Ein Zusatz von solchen Kationen zu gefrorenem und wieder aufgetautem Plasma oder auch zu dem Überstand nach Abtrennung von Kryopräzipitat stabilisiert ebenfalls deutlich den F V.

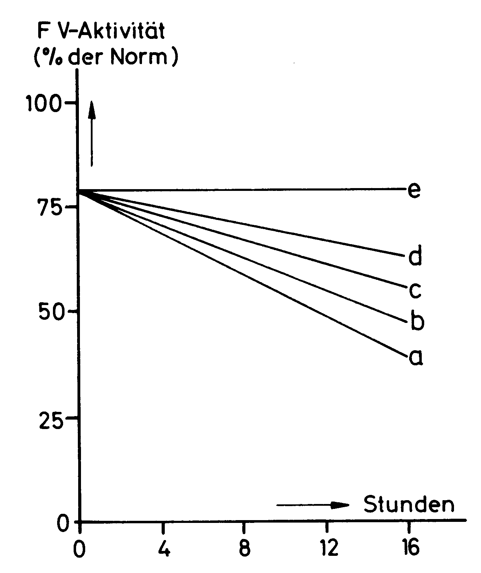

Fig. 1 zeigt die Abhängigkeit der F V-Aktivität von der Zeit und der Calciumkonzentration. Der pH-Wert betrug wie im Citratplasma 7,5-7,8, die Temperatur 4°C.

Dazu wurde humanes Citrat-Gefrierplasma, das durch Einfrieren eines Gemischs aus 9 Volumteilen Vollblut und einem Volumteil Natriumcitrat und mit einer Citratendkonzentration von 10 mmol/l gewonnen wurde, aufgetaut und gegen einen physiologischen Puffer mit einer Citrat-Konzentration von 10 mmol/l und einer Calciumkonzentration von 0,25, 0,5, 1 oder 2 mmol/l dialysiert.

In Fig. 1 entspricht a einer Konzentration von Calcium von 0, b von 0,25, c von 0,5, d von 1 und e von 2 mmol/l. Während bei einer Konzentration von 2 mmol/l Calcium in der citrathaltigen F V-Lösung über 16 Stunden die F V-Aktivität nahezu konstant blieb, fiel diese bei geringeren Calciumkonzentrationen zeitabhängig ab.

Aus dem Voranstehenden ist ersichtlich, daß eine Konzentration von Calciumionen von mindestens 2 mmol $Ca^{2+}$/l in einem Milieu von 10 mmol/l Natriumcitrat ausreichend ist, den F V in einem frisch gewonnnenen Citratplasma, einem Citrat-Gefrierplasma oder einem kryoarmen Citratplasma zu stabilisieren.

Für die Gerinnungsanalytik wird routinemäßig vorwiegend Citratplasma verwendet, zu dessen Gewinnung unter Vorlage einer Tri-Na-Citratlösung Blut aus einer Armvene entnommen wird.

Ein derartiges, partiell rekalzifiziertes Citratplasma ist als Ausgangsmaterial für die Gewinnung eines F V-Konzentrates geeignet.

Wenn frischem Human-Citratplasma (Tri-Na-Citrat-Endkonzentration: 11 mmol/l) 1 USP-E/ml Heparin zugesetzt und diese Lösung bei 20°C gehalten wurde, fiel die F V-Aktivität wesentlich stärker ab als in der Kontrolle ohne Heparinzusatz. Durch Zusatz von 5 mmol/l Calciumchlorid kann dieser Aktivitätsabfall aber auch unter diesen Bedingungen drastisch verringert werden.

Dies zeigt, daß man Citrat mehr oder weniger durch Heparin ersetzen und den F V durch Calcium stabilisieren kann. Man kann also auch auf diese Weise einen antikoagulatorischen Effekt erzielen, ohne dem F V das für seine Stabilisierung notwendige Calcium zu entziehen.

Mit Calcium ist aber nicht nur eine Stabilisierung von F V in Plasma möglich, sondern auch in daraus hergestellten Fraktionen. Dabei können als Antikoagulantien alle die üblicherweise verwendeten benutzt werden.

Als F V-haltige Fraktion, die als Ausgangsmaterial für die Herstellung eines Konzentrats brauchbar ist, kann der Plasmaüberstand nach Abtrennung des Kryopräzipitats verwendet werden. Auch im kryopräzipitat-freien Plasma kann der F V durch Calcium stabilisiert werden. Das gilt auch für die Arbeitsschritte, die zur Gewinnung eines F V-Konzentrates eingesetzt werden können, beispielsweise Chromatographie von einem kryopräzipität-freien Plasma an einem Anionenaustauscher.

F V-Bestimmung: Die F V-Bestimmung kann nach der Methode von Koller, Loeliger und Duckert (Perlick, Gerinnungslaboratorium in Klinik und Praxis, Verlag: VEB Georg Thieme, Leipzig, 1960) durchgeführt werden.

Dazu wird 0,1 ml F V-Mangelplasma mit 0,1 ml einer definierten Verdünnung von Normalplasma vermischt. Diese Mischung wird 30 s bei 37°C inkubiert. Danach fügt man 0,2 ml eines calciumhaltigen Thromboplastins bei und bestimmt die Zeit bis zum Auftreten eines Gerinnsels. Auf diese Weise wird eine Bezugskurve erstellt.

Bei der quantitativen F V-Bestimmung eines zu untersuchenden Plasmas oder Konzentrates werden Verdünnungen davon in Barbitalpuffer anstelle von Normalplasma im oben beschriebenen Test eingesetzt und die Gerinnungszeit bestimmt. Unter Bezugnahme auf eine mit einer Normalplasma-Verdünnungsreihe erhaltenen Eichkurve kann die F V-Konzentration ermittelt werden.

Eine Einheit F V entspricht der F V-Aktivität von 1 ml Normalplasma.

Die folgenden Beispiele erläutern die Erfindung.

Beispiel 1

F V-Stabilisierung in Gefrierplasma

Frisches humanes Citratplasma wurde aus 1 Teil 110 mmol/l Tri-Na-Citrat und 9 Teilen Vollblut durch Abtrennen der Blutzellen gewonnen. Das Plasma wurde bei -25°C eingefroren und nach 24 Stunden wieder aufgetaut, partiel rekalzifiziert und bei 37°C inkubiert. Zu bestimmten Zeiten wurde während der Inkubation die F V-Aktivität gemessen:

Tabelle 1

| F V-Aktivität in aufgetautem Plasma, das ohne Zusätze und mit 2,5 mmol oder 5 mmol/l $Ca^{2+}$ inkubiert wurde. | | | |
|---|---|---|---|
| Inkubationszeit | F V-Restaktivität in % | | |
| | Gefrierplasma ohne Zusätze | Gefrierplasma + 2,5mmol/l $Ca^{2+}$ | Gefrierplasma + 5 mmol/l $Ca^{2+}$ |
| 0 | 100 | 100 | 100 |
| 4 Std. | 59 | 81 | 81 |
| 6 Std. | 46 | 77 | 81 |
| 21 Std. | 10 | 38 | 74 |

Beispiel 2

F V-Stabilisierung während der Gewinnung einer Plasmafraktion aus Citratplasma

4,74 l gefrorenes Human-Citratplasma wurden bei 2-4°C aufgetaut und der ungelöst gebliebene

EP 0 244 834 B1

Rückstand, das Kryopräzipitat, durch Zentrifugation abgetrennt. Zum Überstand wurde zur Entfernung der PPSB-Faktoren bei 15°C 237 ml einer 1 g/100 ml enthaltenden Al(OH)$_3$-Suspension gegeben, 15 min gerührt, der Niederschlag durch Zentrifugation abgetrennt und die Lösung mit 1 USP-E/ml Heparin versetzt.

Die Lösung wurde in 2 Teile von jeweils 2350 ml geteilt. Teil 1 wurde mit soviel CaCl$_2$ versetzt, daß die Konzentration 5 mmol/l betrug, und Teil 2 so belassen. Beide wurden unter identischen Bedingungen zu einem F V-Konzentrat aufgearbeitet, mit dem Unterschied, daß für Teil 1 alle Lösungsmittel, nämlich Äquilibrier-, Wasch- und Dialysemedien, 5 mmol/l CaCl$_2$ enthielten.

Zur Gewinnung des F V wurde zu jedem Teil 32 g feucht gepacktes DEAE-Sephadex$^{(R)}$A 50, das mit einer Lösung, die 60 mmol/l NaCl, 20 mmol/l Tri-Na-Citrat und für Teil 1 zusätzlich 5 mmol/l CaCl$_2$ enthielt, bei pH 7,5 äquilibriert worden war, zugesetzt und 1 Std. bei 15°C gerührt.

Das Anionenaustauscher-Harz wurde vom Überstand getrennt, in eine Säule überführt und mit 1,5 l physiologischer NaCl-Lösung von Plasmaresten freigewaschen.

Die Elution erfolgte mit einer Lösung, die 1 mol/l NaCl, 1 USP-E/ml Heparin, 0,2 E/ml AT III und für Teil 2 weiterhin 5 mmol/l CaCl$_2$ enthielt. Das Eluat wurde in 3 Fraktionen zu 30 ml, 70 ml und 180 ml gesammelt. Zu den Fraktionen 2 und 3 wurde soviel Ammoniumsulfat gegeben, daß 60 % der Sättigung erreicht wurden, der Niederschlag in 5 ml physiologischer Kochsalzlösung gelöst, dialysiert und lyophilisiert.

Während in der Aufarbeitung mit Zusatz von Calciumchlorid die gesamte nach der Aluminiumhydroxid-Adsorption vorhandene F V-Aktivität im Lyophilisat wiedergefunden wurde, waren es ohne Calcium nur 20 % davon.

**Patentansprüche**

1. Verfahren zur Herstellung eines Konzentrats von Gerinnungsfaktor F V aus einem ein Antikoagulans enthaltenden humanen Blutplasma oder Blut, dadurch gekennzeichnet, daß man einem solchen Plasma oder Blut ein Salz von Calcium, Magnesium oder Mangan in einer Menge zusetzt, daß eine Konzentration an zweiwertigen Kationen dieser Metalle von mindestens 2 mmol/l aber kleiner als die der Konzentration an Antikoagulans äquivalenten Konzentration erreicht wird, und auf bekannte Weise ein F V-Konzentrat gewinnt.

2. Verfahren nach Anspruch 1, dadurch gekennzeichnet, daß ein lösliches Salz des Calciums, besonders Calciumchlorid oder -azetat zugesetzt wird.

3. Verfahren nach Anspruch 1, dadurch gekennzeichnet, daß das Antikoagulans Citrat, Heparin, Phosphatdextrose oder Citratdextrose oder eine Mischung solcher Antikoagulantien ist.

4. Verfahren nach Anspruch 1, dadurch gekennzeichnet, daß das Antikoagulans Heparin ist.

5. Verfahren nach Anspruch 1, dadurch gekennzeichnet, daß das Antikoagulans ein Gemisch aus einem Salz der Zitronensäure und Heparin ist.

**Claims**

1. A process for the preparation of a concentrate of coagulation factor F V from an anticoagulant-containing human blood plasma or blood, which comprises addition to such a plasma or blood of a salt of calcium, magnesium or manganese in an amount such that the concentration achieved of doubly charged cations of these metals is at least two mmol/l but less than the concentration equivalent to the concentration of anticoagulant, and an F V concentrate being obtained in known manner.

2. The process as claimed in claim 1, wherein a soluble salt of calcium, in particular calcium chloride or acetate, is added.

3. The process as claimed in claim 1, wherein the anticoagulant is citrate, heparin, phosphate-dextrose or citrate-dextrose or a mixture of such anticoagulants.

4. The process as claimed in claim 1, wherein the anticoagulant is heparin.

5. The process as claimed in claim 1, wherein the anticoagulant is a mixture of a salt of citric acid and

6

heparin.

**Revendications**

1. Procédé pour la préparation d'un concentré de facteur de coagulation F V à partir d'un sang ou plasma sanguin humain contenant un anticoagulant, caractérisé en ce que l'on ajoute à un tel sang ou plasma un sel de calcium, magnésium ou manganèse en une quantité telle qu'est atteinte une concentration de cations bivalents de ces métaux d'au moins 2 mmoles/l mais inférieure à la concentration équivalente à la concentration d'anticoagulant, et on obtient de façon connue un concentré de F V.

2. Procédé selon la revendication 1, caractérisé en ce que l'on ajoute un sel soluble du calcium, en particulier le chlorure ou l'acétate de calcium.

3. Procédé selon la revendication 1, caractérisé en ce que l'anticoagulant est le citrate, l'héparine, le D-glucose phosphaté ou le D-glucose citraté ou un mélange de ces anticoagulants.

4. Procédé selon la revendication 1, caractérisé en ce que l'anticoagulant est l'héparine.

5. Procédé selon la revendication 1, caractérisé en ce que l'anticoagulant est un mélange d'un sel de l'acide citrique et d'héparine.